# EUROPEAN PATENT SPECIFICATION

(11) **EP 4 166 090 B1**
(45) Date of publication and mention of the grant of the patent: **28.01.2026**
(21) Application number: 21826235.0
(22) Date of filing: 10.03.2021
(51) Int. Cl.: A61B 8/08, A61B 8/14, A61B 8/00

(54) **ULTRASONIC DIAGNOSTIC APPARATUS, CONTROL METHOD FOR ULTRASONIC DIAGNOSTIC APPARATUS, AND PROCESSOR FOR ULTRASONIC DIAGNOSTIC APPARATUS**
ULTRASCHALLDIAGNOSEVORRICHTUNG, STEUERUNGSVERFAHREN FÜR DIE ULTRASCHALLDIAGNOSEVORRICHTUNG UND PROZESSOR FÜR DIE ULTRASCHALLDIAGNOSEVORRICHTUNG
APPAREIL DE DIAGNOSTIC ULTRASONORE, PROCÉDÉ DE COMMANDE D'UN APPAREIL DE DIAGNOSTIC ULTRASONORE ET PROCESSEUR D'UN APPAREIL DE DIAGNOSTIC ULTRASONORE

(30) Priority: 15.06.2020 JP 2020102937
(43) Date of publication of application: 19.04.2023
(73) Proprietor: FUJIFILM Corporation, Tokyo 106-8620 (JP)
(72) Inventor: KARUBE Mikihiko, Ashigarakami-gun, Kanagawa 258-8538 (JP)
(74) Representative: Dehns Germany Partnerschaft mbB
(86) International application number: PCT/JP2021/009605
(87) International publication number: WO 2021/256019

(56) References cited:
- JP-A- 2016 224 396
- JP-A- 2017 012 427
- US-A1- 2015 133 784
- US-A1- 2016 100 790
- US-A1- 2018 132 722
- US-A1- 2019 050 991
- PORTER-ARMSTRONG ALISON P. ET AL: "Do High Frequency Ultrasound Images Support Clinical Skin Assessment?", vol. 2013, 21 February 2013 (2013-02-21), pages 1 - 5, XP093091654, Retrieved from the Internet <URL:https://downloads.hindawi.com/archive/2013/314248.pdf?_gl=1*ci9qmq*_ga*MTQzMDc5OTcwOC4xNjk3MjA5Njcw*_ga_NF5QFMJT5V*MTY5NzIwOTY3MC4xLjAuMTY5NzIwOTY3MC42MC4wLjA.&_ga=2.82189653.235055227.1697209670-1430799708.1697209670> DOI: 10.1155/2013/314248
- YABUNAKA KOICHI ET AL: "Three-dimensional ultrasound imaging of the pressure ulcer. A case report", vol. 17, no. 3, 1 September 2015 (2015-09-01), pages 404, XP093090179, ISSN: 1844-4172, Retrieved from the Internet <URL:http://medultrason.ro/medultrason/index.php/medultrason/article/viewFile/683/664> DOI: 10.11152/mu.2013.2066.173.kya
- NIJMA AYA: "An example of bedsore which ultrasonography was useful for diagnosis", JOURNAL OF MEDICAL ULTRASONICS, vol. 43, 30 November 2015 (2015-11-30), pages S864, XP009540880, ISSN: 1346-1176, DOI: 10.3179/jjmu.JJMU.S.107

## Description

### BACKGROUND OF THE INVENTION

### 1. Field of the Invention

The present invention relates to an ultrasound diagnostic apparatus, a method for controlling an ultrasound diagnostic apparatus, and a processor for an ultrasound diagnostic apparatus for observing a decubitus of a subject.

### 2. Description of the Related Art

In the related art, a decubitus occurring in a subject has been observed using an ultrasound diagnostic apparatus. For example, as disclosed in JP2016-224396A, in general, since a decubitus may extend to a deep part of a subject, a user moves an ultrasound probe in a plurality of directions to capture ultrasound images in order to understand the distribution of the decubitus. US 2015/133784 A1 relates to three-dimensional ultrasound reconstruction with confidence information. US 2019/050991 A1 relates to a method and a system for assessing burn wound depth. US 2018/132722 A1 relates to a method, an apparatus, and a system for complete examination of tissue with hand-held imaging devices. PORTER-ARMSTRONG ALISON P. ET AL: "Do High Frequency Ultrasound Images Support Clinical Skin Assessment?", vol. 2013 21 February 2013 (2013-02-21), pages 1-5,relates to high frequency ultrasound imaging for skin assessment. Further, YABUNAKA KOICHI ET AL: "Three-dimensional ultrasound imaging of the pressure ulcer. A case report",MEDICAL ULTRASONOGRAPHY,vol. 17, no. 3 1 September 2015 (2015-09-01), refers to ultrasound imaging of pressure ulcers.

### SUMMARY OF THE INVENTION

However, in general, the decubitus has a three-dimensional spread in the subject. In many cases, a plurality of findings with different progressions of symptoms are mixed in the decubitus. Therefore, in particular, it is difficult for users with a low level of skill to check the captured ultrasound images and to accurately understand the type of finding of the decubitus and the three-dimensional spread of the decubitus. In addition, for example, in a case in which the decubitus is large, a case in which a bone protrudes in a region with which an ultrasound probe is brought into contact and it is difficult to normally bring the ultrasound probe into contact with a body surface, and a case in which the decubitus spreads to a portion that is not capable of being determined only by checking of the body surface of the subject by the user, it may be difficult to accurately understand the type of finding of the decubitus and the three-dimensional spread of the decubitus.

The invention has been made in order to solve this problem of the related art, and an object of the invention is to provide an ultrasound diagnostic apparatus, a method for controlling an ultrasound diagnostic apparatus, and a processor for an ultrasound diagnostic apparatus that enable a user to accurately understand the type of finding of a decubitus and a three-dimensional distribution of the decubitus.

In order to achieve the above-described object, according to the invention, there is provided an ultrasound diagnostic apparatus according to claim 1.

The probability calculation unit may calculate a plurality of the presence probabilities corresponding to each of a plurality of predetermined findings, and the probability map generation unit may three-dimensionally visualize each of the plurality of presence probabilities and generate a three-dimensional probability map, in which the plurality of findings have been integrated, on the basis of the visualized plurality of presence probabilities.

The ultrasound diagnostic apparatus may further comprise a monitor that displays the probability map.

In this case, the ultrasound diagnostic apparatus may further comprise a finding information calculation unit that calculates finding information including at least one of a size, a depth, or a volume of the finding on the basis of the probability map, and the monitor may display the finding information.

In addition, the probability map generation unit may generate the probability map in which a shade of a color is changed according to a value of the presence probability calculated by the probability calculation unit.

Further, in a case in which a plurality of scanning operations are performed on the same wound portion, the probability map generation unit may generate the probability map on the basis of a plurality of the presence probabilities calculated by the probability calculation unit corresponding to the plurality of scanning operations.

Alternatively, in a case in which a plurality of scanning operations are performed on the same wound portion, the probability map generation unit may generate the probability map for each of the scanning operations, integrate a plurality of the probability maps generated corresponding to the plurality of scanning operations, and display an integrated probability map on the monitor.

The ultrasound diagnostic apparatus may further comprise a failure region detection unit that analyzes the probability map or the ultrasound image to detect a failure region in which the probability map is not normally generated or the ultrasound image is not normally acquired. The monitor may display the failure region.

In addition, the ultrasound diagnostic apparatus may further comprise a scanned region detection unit that detects that a region has already been scanned on the basis of the positional information of the ultrasound probe acquired by the position sensor in a case in which scanning is performed on the wound portion; and a notification unit that notifies a user in a case in which it is detected that the region has already been scanned.

The monitor may display the probability map that has already been generated by the probability map generation unit on the basis of the positional information of the ultrasound probe acquired by the position sensor in a case in which the ultrasound diagnostic apparatus scans the wound portion.

In addition, the probability map generation unit may generate the probability map in which a region, which has been repeatedly scanned a larger number of times, has a darker color or a higher density.

According to the invention, there is provided a method for controlling an ultrasound diagnostic apparatus according to claim 12.

According to the invention, there is provided a processor for an ultrasound diagnostic apparatus according to claim 13.

According to the invention, the ultrasound diagnostic apparatus comprises the position sensor that is attached to the ultrasound probe and acquires the positional information of the ultrasound probe, the probability calculation unit that calculates the presence probability of a finding related to a wound portion from each of the ultrasound images of a plurality of frames, and the probability map generation unit that generates the three-dimensional probability map of the finding on the basis of the positional information of the ultrasound probe acquired by the position sensor and the presence probability calculated by the probability calculation unit. Therefore, the user can accurately understand the type of finding of a decubitus and the three-dimensional distribution of the decubitus.

### BRIEF DESCRIPTION OF THE DRAWINGS

Fig. 1 is a block diagram illustrating a configuration of an ultrasound diagnostic apparatus according to Embodiment 1 of the invention.
Fig. 2 is a block diagram illustrating an internal configuration of a transmitting and receiving circuit in Embodiment 1 of the invention.
Fig. 3 is a block diagram illustrating an internal configuration of an image generation unit according to Embodiment 1 of the invention.
Fig. 4 is a diagram schematically illustrating an unclear layer structure.
Fig. 5 is a diagram schematically illustrating a cobblestone-like pattern.
Fig. 6 is a diagram schematically illustrating a cloud-like pattern.
Fig. 7 is a diagram schematically illustrating a pattern in which liquid accumulation is observed.
Fig. 8 is a diagram schematically illustrating a cross-sectional view of an integrated probability map.
Fig. 9 is a diagram schematically illustrating a failure region.
Fig. 10 is a flowchart illustrating an operation of the ultrasound diagnostic apparatus according to Embodiment 1 of the invention.
Fig. 11 is a diagram schematically illustrating an ultrasound probe that scans a wound portion along a predetermined direction in Embodiment 1 of the invention.
Fig. 12 is a flowchart illustrating an operation of an ultrasound diagnostic apparatus according to Embodiment 2 of the invention.
Fig. 13 is a diagram schematically illustrating an ultrasound probe that scans a wound portion a plurality of times in a predetermined direction in Embodiment 2 of the invention.
Fig. 14 is a flowchart illustrating an operation of an ultrasound diagnostic apparatus according to Embodiment 3 of the invention.
Fig. 15 is a diagram schematically illustrating an ultrasound probe that scans a wound portion in two directions in Embodiment 3 of the invention.
Fig. 16 is a flowchart illustrating an operation of an ultrasound diagnostic apparatus according to Embodiment 4 of the invention.
Fig. 17 is a flowchart illustrating an operation of an ultrasound diagnostic apparatus according to Embodiment 5 of the invention.
Fig. 18 is a flowchart illustrating an operation of an ultrasound diagnostic apparatus according to Embodiment 6 of the invention.
Fig. 19 is a block diagram illustrating a configuration of an ultrasound diagnostic apparatus according to Embodiment 7 of the invention.

### DESCRIPTION OF THE PREFERRED EMBODIMENTS

Hereinafter, embodiments of the invention will be described with reference to the accompanying drawings.

The following description of components is based on a representative embodiment of the invention. However, the invention is not limited to the embodiment.

In addition, in the specification, a numerical range represented by "to" means a range including numerical values described before and after "to" as a lower limit value and an upper limit value.

In the specification, the terms "same" and "similar" include an error range generally allowed in the technical field.

### Embodiment 1

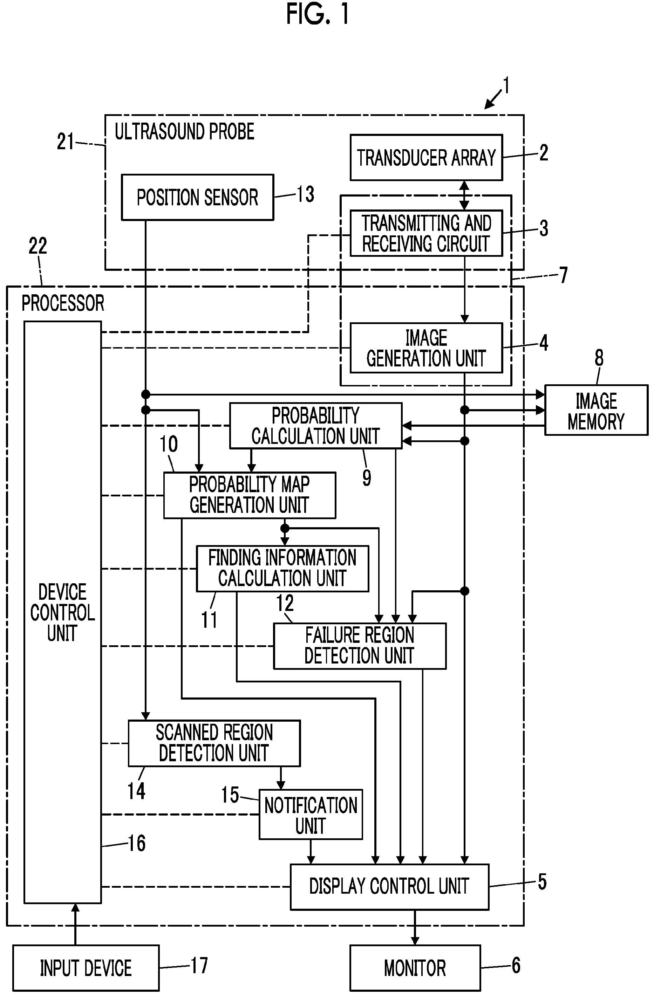

Fig. 1 illustrates a configuration of an ultrasound diagnostic apparatus 1 according to Embodiment 1 of the invention. The ultrasound diagnostic apparatus 1 comprises a transducer array 2, and a transmitting and receiving circuit 3, an image generation unit 4, a display control unit 5, and a monitor 6 are sequentially connected to the transducer array 2. The transducer array 2 and the transmitting and receiving circuit 3 are included in an ultrasound probe 21. In addition, the transmitting and receiving circuit 3 and the image generation unit 4 constitute an image acquisition unit 7. An image memory 8 is connected to the image generation unit 4. In addition, a probability calculation unit 9 is connected to the image generation unit 4 and the image memory 8. A probability map generation unit 10 and a finding information calculation unit 11 are sequentially connected to the probability calculation unit 9. The probability map generation unit 10 and the finding information calculation unit 11 are connected to the display control unit 5. Further, a failure region detection unit 12 is connected to the probability calculation unit 9 and the probability map generation unit 10. The failure region detection unit 12 is connected to the display control unit 5. Furthermore, a position sensor 13 is attached to the ultrasound probe 21, and a scanned region detection unit 14 and a notification unit 15 are sequentially connected to the position sensor 13. Moreover, the position sensor 13 is connected to the image memory 8. In addition, the notification unit 15 is connected to the display control unit 5.

Further, a device control unit 16 is connected to the transmitting and receiving circuit 3, the image generation unit 4, the display control unit 5, the probability calculation unit 9, the probability map generation unit 10, the finding information calculation unit 11, the failure region detection unit 12, the scanned region detection unit 14, and the notification unit 15. Furthermore, an input device 17 is connected to the device control unit 16.

In addition, the image generation unit 4, the display control unit 5, the probability calculation unit 9, the probability map generation unit 10, the finding information calculation unit 11, the failure region detection unit 12, the scanned region detection unit 14, the notification unit 15, and the device control unit 16 constitute a processor 22 for the ultrasound diagnostic apparatus 1.

The transducer array 2 of the ultrasound probe 21 illustrated in Fig. 1 has a plurality of transducers that are one-dimensionally or two-dimensionally arranged. Each of the transducers transmits ultrasonic waves in response to a driving signal supplied from the transmitting and receiving circuit 3. In addition, each of the transducers receives ultrasound echoes from a subject and outputs a signal based on the ultrasound echoes. For example, each transducer is configured by forming electrodes at both ends of a piezoelectric body consisting of piezoelectric ceramic typified by lead zirconate titanate (PZT), a polymer piezoelectric element typified by polyvinylidene difluoride (PVDF), or a piezoelectric single crystal typified by lead magnesium niobate-lead titanate (PMN-PT).

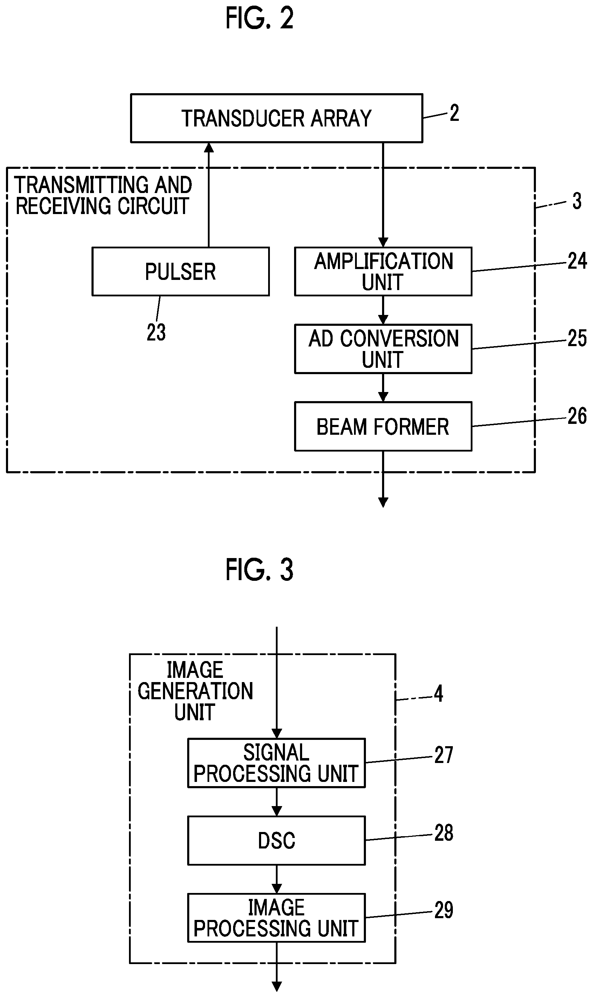

The transmitting and receiving circuit 3 transmits ultrasonic waves from the transducer array 2 and generates a sound ray signal on the basis of a received signal acquired by the transducer array 2 under the control of the device control unit 16. As illustrated in Fig. 2, the transmitting and receiving circuit 3 includes a pulser 23 that is connected to the transducer array 2, and an amplification unit 24, an analog-digital (AD) conversion unit 25, and a beam former 26 that are sequentially connected in series to the transducer array 2.

The pulser 23 includes, for example, a plurality of pulse generators and supplies each driving signal to the plurality of transducers while adjusting the amount of delay such that the ultrasonic waves transmitted from the plurality of transducers of the transducer array 2 form an ultrasound beam, on the basis of a transmission delay pattern selected in response to a control signal from the device control unit 16. As described above, in a case in which a pulsed or continuous-wave voltage is applied to the electrodes of the transducers of the transducer array 2, the piezoelectric body is expanded and contracted and pulsed or continuous ultrasonic waves are generated from each transducer. An ultrasound beam is formed from a combined wave of the ultrasonic waves.

The transmitted ultrasound beam is reflected from a target, such as a part of the subject, and is propagated toward the transducer array 2 of the ultrasound probe 21. The ultrasound echoes propagated toward the transducer array 2 in this way are received by each transducer constituting the transducer array 2. In this case, each of the transducers constituting the transducer array 2 receives propagated ultrasound echoes, is expanded and contracted to generate a received signal which is an electric signal, and outputs the received signal to the amplification unit 24.

The amplification unit 24 amplifies the signal input from each of the transducers constituting the transducer array 2 and transmits the amplified signal to the AD conversion unit 25. The AD conversion unit 25 converts the signal transmitted from the amplification unit 24 into digital reception data and transmits the reception data to the beam former 26. The beam former 26 performs a so-called reception focus process by giving a delay to each reception data item converted by the AD conversion unit 25 according to a sound speed or a sound speed distribution set on the basis of a reception delay pattern selected in response to a control signal from the device control unit 16 and adding each reception data item. Each reception data item converted by the AD conversion unit 25 is phase-adjusted and added, and a sound ray signal in which the focus of the ultrasound echo has been narrowed down is acquired by this reception focus process.

As illustrated in Fig. 3, the image generation unit 4 has a configuration in which a signal processing unit 27, a digital scan converter (DSC) 28, and an image processing unit 29 are sequentially connected in series to each other.

The signal processing unit 27 corrects the attenuation of the sound ray signal generated by the beam former 26 of the transmitting and receiving circuit 3 caused by a distance according to the depth of the position where the ultrasonic waves are reflected and then performs an envelope detection process on the sound ray signal to generate a B-mode image signal which is tomographic image information related to the tissues in the subject.

The DSC 28 converts the B-mode image signal generated by the signal processing unit 27 into an image signal following a normal television signal scanning method (raster conversion).

The image processing unit 29 performs various types of necessary image processing including a gradation process on the B-mode image signal input from the DSC 28 and then outputs the B-mode image signal to the display control unit 5 and the image memory 8. Hereinafter, the B-mode image signal subjected to the image processing by the image processing unit 29 is simply referred to as an ultrasound image.

The image memory 8 is a memory that stores a series of ultrasound images of a plurality of frames generated for each diagnosis by the image generation unit 4. For example, a recording medium, such as a hard disk drive (HDD), a solid state drive (SSD), a flexible disc (FD), a magneto-optical disc (MO), a magnetic tape (MT), a random access memory (RAM), a compact disc (CD), a digital versatile disc (DVD), a secure digital card (SD card), or a universal serial bus memory (USB memory), or a server can be used as the image memory 8.

In a case in which the image generation unit 4 generates an ultrasound image of a tomographic plane including a so-called decubitus wound portion, the probability calculation unit 9 performs image recognition on each of the ultrasound images of the plurality of frames generated by the image generation unit 4 to calculate the presence probability of findings related to the wound portion. Here, the wound portion means a portion where a decubitus occurs and a region around the portion.

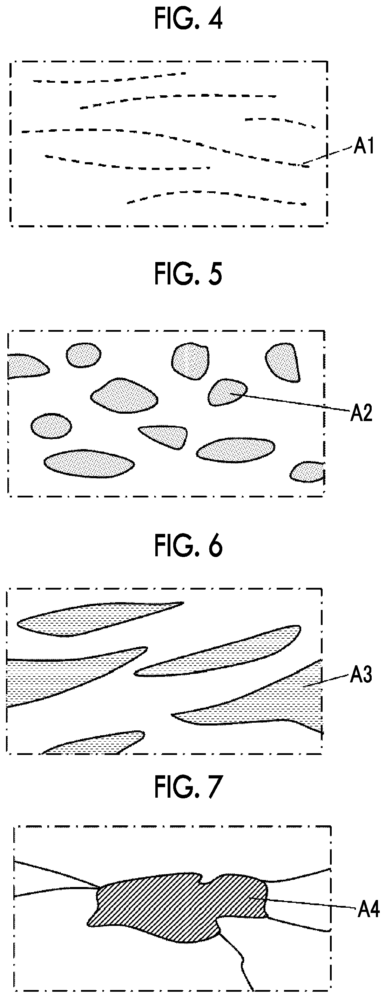

In addition, the findings related to the wound portion mean the structure and pattern of the tissues of the subject corresponding to symptoms related to the decubitus such as edema, necrosis, and abscess. Examples of the types of findings include an unclear layer structure A1 illustrated in Fig. 4, a cobblestone-like pattern A2 illustrated in Fig. 5, a cloud-like pattern A3 illustrated in Fig. 6, and a pattern A4 illustrated in Fig. 7 which has low brightness and in which liquid accumulation is recognized. The unclear layer structure A1 illustrated in Fig. 4 corresponds to weak edema, the cobblestone-like pattern A2 illustrated in Fig. 5 corresponds to strong edema, the cloud-like pattern A3 illustrated in Fig. 6 corresponds to suspected necrosis, and the pattern A4 illustrated in Fig. 7 in which liquid accumulation is recognized corresponds to suspected abscess, hematoma, or edema.

In addition, the probability calculation unit 9 can calculate the presence probabilities of a plurality of findings for each pixel of the ultrasound image of each frame, using a deep learning method such as so-called U-net. In this case, for example, the following are calculated for one pixel: the probability that the pixel will correspond to the unclear layer structure A1; the probability that the pixel will correspond to the cobblestone-like pattern A2; the probability that the pixel will correspond to the cloud-like pattern A3; and the probability that the pixel will correspond to the pattern A4 in which liquid accumulation is recognized.

The position sensor 13 attached to the ultrasound probe 21 acquires the positional information of the ultrasound probe 21. The position sensor 13 can be composed of, for example, a so-called acceleration sensor, a gyro sensor, and the like.

The probability map generation unit 10 generates a probability map indicating a three-dimensional presence distribution of each finding on the basis of the positional information of the ultrasound probe 21 acquired by the position sensor 13 and the presence probabilities of the plurality of findings calculated by the probability calculation unit 9.

For example, the probability map generation unit 10 can generate a three-dimensional probability map of each finding by three-dimensionally plotting the presence probability of each finding calculated by the probability calculation unit 9 for each of the plurality of findings on the basis of the positional information of the ultrasound probe 21 acquired by the position sensor 13 to visualize the presence probability of each finding.

Here, the three-dimensional probability map of each finding is information in which the presence probability of each finding for each of the pixels three-dimensionally disposed is plotted, such as information indicating the three-dimensional presence probability distribution of the unclear layer structure A1 illustrated in Fig. 4, information indicating the three-dimensional presence probability distribution of the cobblestone-like pattern A2 illustrated in Fig. 5, information indicating the three-dimensional presence probability distribution of the cloud-like pattern A3 illustrated in Fig. 6, and information indicating the three-dimensional presence probability distribution of the pattern A4 illustrated in Fig. 7 in which liquid accumulation is recognized, and indicates the three-dimensional presence probability distribution of each finding.

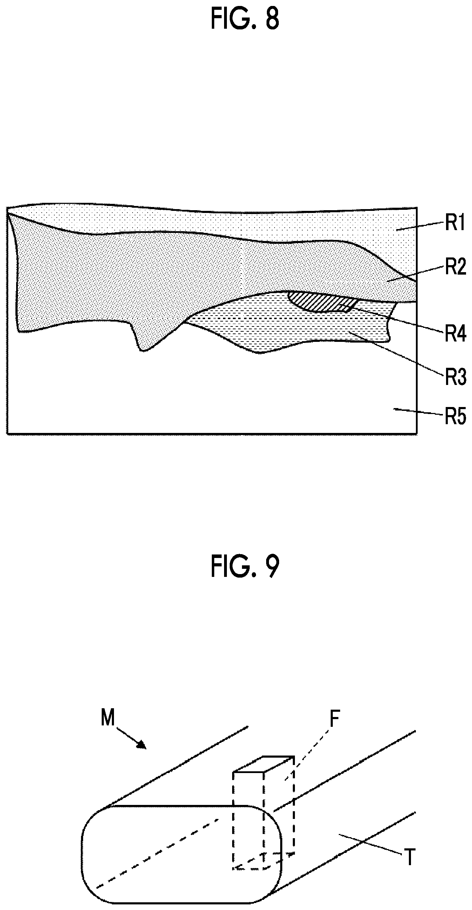

In addition, the probability map generation unit 10 integrates the generated three-dimensional probability maps of the plurality of findings to generate, for example, an integrated probability map having a cross section illustrated in Fig. 8. The integrated probability map illustrated in Fig. 8 has a region R1 corresponding to the unclear layer structure A1, a region R2 corresponding to the cobblestone-like pattern A2, a region R3 corresponding to the cloud-like pattern A3, a region R4 corresponding to the pattern A4 in which liquid accumulation is recognized, and a background region R5 that does not correspond to any findings.

In a case in which the probability maps of each finding are integrated, the probability map generation unit 10 assigns one finding to each pixel on the basis of the value of the presence probability of each finding plotted for each pixel. For example, in a case in which one pixel has four presence probabilities of the presence probability of the unclear layer structure A1, the presence probability of the cobblestone-like pattern A2, the presence probability of the cloud-like pattern A3, and the presence probability of the pattern A4 in which liquid accumulation is recognized, the probability map generation unit 10 can assign a finding corresponding to the highest presence probability among the four presence probabilities to one pixel.

In addition, the probability map generation unit 10 displays the obtained integrated probability map on the monitor 6. For example, the probability map generation unit 10 can display the regions R1 to R4 corresponding to the plurality of findings in different colors.

The finding information calculation unit 11 calculates finding information including at least one of the size, depth, or volume of the finding on the basis of the three-dimensional probability map of each of the plurality of findings or the three-dimensional integrated probability map in which the plurality of findings have been integrated. For example, assuming that a depth direction from a body surface of the subject is the Z direction, a direction orthogonal to the Z direction is the X direction, and a direction orthogonal to the X direction and the Z direction is the Y direction, the finding information calculation unit 11 can calculate the maximum width of a region occupied by each finding in the X direction, the maximum width of the region in the Y direction, and the maximum width of the region in the Z direction as the size of the finding. In addition, for example, the finding information calculation unit 11 calculates, as the depth of a fining, a distance from the body surface of the subject to the shallowest portion of each finding or a distance from the body surface of the subject to the deepest portion of each finding. The finding information calculation unit 11 can display the calculated finding information on the monitor 6.

The failure region detection unit 12 analyzes the three-dimensional probability map of each finding or the three-dimensional integrated probability map, in which the plurality of findings have been integrated, generated by the probability map generation unit 10 to detect a three-dimensional failure region in which the probability map has not been generated normally and displays the detected failure region on the monitor 6 to be superimposed on the integrated probability map.

For example, in a case in which a hollow region F located to penetrate a region T corresponding to any one of a plurality of findings in the depth direction occurs in a three-dimensional probability map M as illustrated in Fig. 9 because the ultrasound probe 21 is separated from the body surface of the subject and an ultrasound image indicating the tomographic plane of the subject is not acquired, the failure region detection unit 12 can detect the hollow region F as the failure region.

Further, for example, in a case in which the probability calculation unit 9 calculates the presence probability of the background region R5 that does not correspond to the plurality of findings, the failure region detection unit 12 can detect, as the failure region, a region that is surrounded by a region corresponding to any one of the plurality of findings and has been detected as the background region R5.

In a case in which the user scans the wound portion of the subject, the scanned region detection unit 14 detects that the region has already been scanned in the same examination on the same subject on the basis of the positional information of the ultrasound probe 21 acquired by the position sensor 13. For example, the scanned region detection unit 14 stores the positional information of the ultrasound probe 21 in the same examination, collates the stored positional information of the ultrasound probe 21 with the positional information of the ultrasound probe 21 newly obtained by the position sensor 13, and detects that the region currently being scanned by the ultrasound probe 21 is the region that has already been scanned in a case in which the two positional information items are matched with each other.

In a case in which the scanned region detection unit 14 detects that the region currently being scanned by the ultrasound probe 21 is the region that has already been scanned, the notification unit 15 notifies the user of the fact. For example, the notification unit 15 can display a message indicating that the region currently being scanned by the ultrasound probe 21 is the region that has already been scanned on the monitor 6 to notify the user of the fact.

The device control unit 16 controls each unit of the ultrasound diagnostic apparatus 1 on the basis of, for example, a control program stored in advance.

The input device 17 is used by the user to perform an input operation and can be configured to comprise, for example, a keyboard, a mouse, a trackball, a touch pad, and a touch panel.

The display control unit 5 performs a predetermined process on the ultrasound images of the frames stored in the image memory 8, the three-dimensional probability map of each finding generated by the probability map generation unit 10, the finding information calculated by the finding information calculation unit 11, the failure region detected by the failure region detection unit 12, and the information indicating the notification to the user by the notification unit 15 and displays the results of the process on the monitor 6 under the control of the device control unit 16.

The monitor 6 displays various kinds of information under the control of the display control unit 5. For example, the monitor 6 includes a display device such as a liquid crystal display (LCD) or an organic electroluminescence (EL) display.

In addition, the processor 22 including the image generation unit 4, the display control unit 5, the probability calculation unit 9, the probability map generation unit 10, the finding information calculation unit 11, the failure region detection unit 12, the scanned region detection unit 14, the notification unit 15, and the device control unit 16 is composed of a central processing unit (CPU) and a control program for causing the CPU to perform various processes. However, the processors may be composed of a field programmable gate array (FPGA), a digital signal processor (DSP), an application specific integrated circuit (ASIC), a graphics processing unit (GPU), and other integrated circuits (ICs) or may be composed of combinations thereof.

In addition, the image generation unit 4, the display control unit 5, the probability calculation unit 9, the probability map generation unit 10, the finding information calculation unit 11, the failure region detection unit 12, the scanned region detection unit 14, the notification unit 15, and the device control unit 16 of the processor 22 may be configured to be partially or wholly integrated into, for example, one CPU.

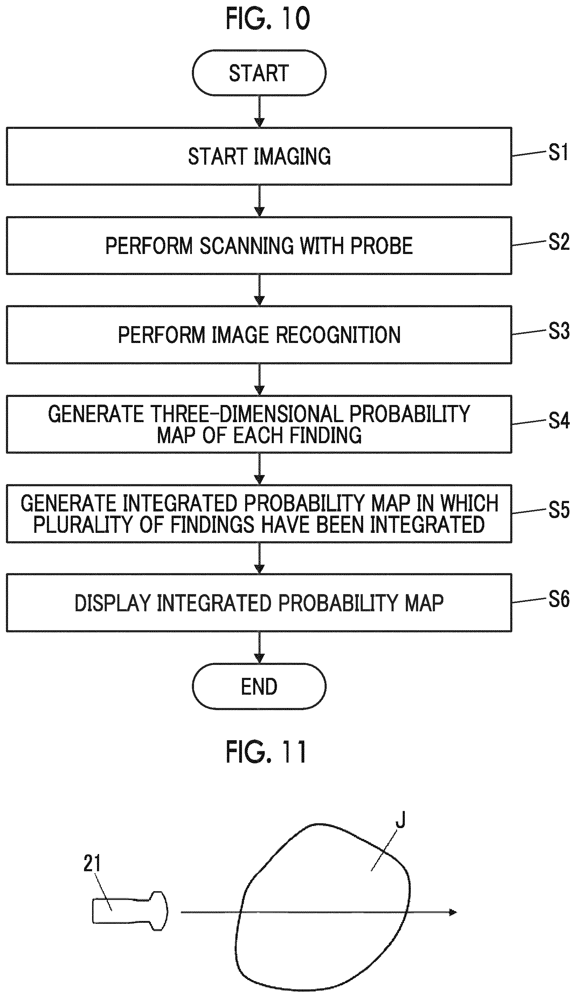

Hereinafter, a basic operation of the ultrasound diagnostic apparatus 1 according to Embodiment 1 will be described in detail with reference to a flowchart illustrated in Fig. 10.

First, in Step S1, the user disposes the ultrasound probe 21 on the body surface of the subject in order to capture an ultrasound image of the wound portion. In this state, the user gives an instruction to start the capture of the ultrasound image through the input device 17. In a case in which the instruction from the user is received, the capture of the ultrasound image is started.

In Step S2, a plurality of transducers of the transducer array 2 transmit ultrasound beams into the subject from in response to a driving signal from the pulser 23 of the transmitting and receiving circuit 3. Each of the transducers that have received ultrasound echoes from the subject output a received signal to the amplification unit 24 of the transmitting and receiving circuit 3. The amplification unit 24 amplifies the received signal, and the AD conversion unit 25 performs AD conversion on the amplified signal. Then, the beam former 26 performs phase adjustment and addition on the converted signal to generate a sound ray signal. In the image generation unit 4, the signal processing unit 27 performs the envelope detection process on this sound ray signal to generate a B-mode image signal. The B-mode image signal is output to the display control unit 5 through the DSC 28 and the image processing unit 29. Then, an ultrasound image is displayed on the monitor 6 under the control of the display control unit 5.

In a state in which the ultrasound images of a plurality of frames are captured and displayed in this way, the user moves the ultrasound probe 21 in parallel only once along a predetermined direction as schematically illustrated in Fig. 11. Therefore, ultrasound images of a plurality of frames of a wound portion J are acquired. The acquired ultrasound images of the plurality of frames are stored in the image memory 8.

In addition, while the ultrasound probe 21 is being moved in parallel by the user, the position sensor 13 acquires the positional information of the ultrasound probe 21 at any time. The acquired positional information is stored in the image memory 8 together with the ultrasound image to be associated with the ultrasound image of the frame acquired at the same timing.

For example, in a case in which the user gives an instruction to end the scanning through the input device 17 after the ultrasound images of the plurality of frames of the wound portion J are acquired and stored in the image memory 8 as described above, the scanning ends.

In Step S3, the probability calculation unit 9 performs image recognition on the ultrasound images of the plurality of frames stored in the image memory 8 to calculate the presence probabilities of the findings related to the wound portion J. The probability calculation unit 9 can calculate the presence probabilities of a plurality of findings for each pixel of the ultrasound images of the plurality of frames using, for example, a deep learning method such as so-called U-net. Therefore, for example, each pixel of the ultrasound images of the plurality of frames has the presence probabilities of a plurality of findings, such as the probability that the pixel will correspond to the unclear layer structure A1, the probability that the pixel will correspond to the cobblestone-like pattern A2, the probability that the pixel will correspond to the cloud-like pattern A3, and the probability that the pixel will correspond to the pattern A4 in which liquid accumulation is recognized, as illustrated in Figs. 4 to 7.

In Step S4, the probability map generation unit 10 generates the three-dimensional probability map of each finding on the basis of the positional information of the ultrasound probe 21 acquired by the position sensor 13 and the presence probabilities of the plurality of findings calculated by the probability calculation unit 9.

In this case, the probability map generation unit 10 can generate the three-dimensional probability map of each finding by plotting the presence probabilities of the plurality of findings obtained in Step S3 for each of the pixels, which are three-dimensionally disposed, on the basis of the positional information of the ultrasound probe 21 stored together with the ultrasound images of the plurality of frames to visualize the presence probability of each finding.

Then, in Step S5, the probability map generation unit 10 integrates the three-dimensional probability maps of each finding generated in Step S4 to generate a three-dimensional integrated probability map. In this case, the probability map generation unit 10 assigns one finding to each pixel on the basis of the value of the presence probability of each finding plotted for each pixel in Step S4. For example, in a case in which one pixel has four presence probabilities of the presence probability of the unclear layer structure A1, the presence probability of the cobblestone-like pattern A2, the presence probability of the cloud-like pattern A3, and the presence probability of the pattern A4 in which liquid accumulation is recognized, the probability map generation unit 10 can assign a finding corresponding to the highest presence probability among the four presence probabilities to one pixel.

In this way, for example, the three-dimensional integrated probability map having the cross section illustrated in Fig. 8 is obtained. The integrated probability map illustrated in Fig. 8 includes the regions R1 to R4 corresponding to four findings of the unclear layer structure A1, the cobblestone-like pattern A2, the cloud-like pattern A3, and the pattern A4 in which liquid accumulation is recognized and the background region R5 that does not correspond to any findings.

Finally, in Step S6, the probability map generation unit 10 displays the integrated probability map obtained in Step S5 on the monitor 6. In this case, for example, the probability map generation unit 10 can display the regions R1 to R4 corresponding to a plurality of findings on the monitor 6 in different colors such that the user can easily check the presence distribution of each finding.

However, in general, a decubitus has a three-dimensional spread in the subject and may have a plurality of findings in which the progression of symptoms is different. In some cases, only the checking of the body surface of the subject makes it difficult for an examiner to determine how spread and distributed the decubitus is. Therefore, in some cases, the decubitus is observed using the ultrasound diagnostic apparatus according to the related art in order to observe the inside of the subject. For example, in a case in which the decubitus is large or in a case in which the ultrasound probe is not capable of being normally brought into contact with the body surface of the subject due to the protrusion of bones in a region with which the ultrasound probe is brought into contact, it may be difficult for the user to accurately understand the type of finding of the decubitus and the three-dimensional spread of the decubitus.

According to the ultrasound diagnostic apparatus 1 of Embodiment 1 of the invention, the three-dimensional probability maps of a plurality of findings of the decubitus are generated, and the generated probability maps are displayed on the monitor 6. Therefore, the user can easily understand the type of finding of the decubitus and the three-dimensional distribution of the decubitus with high accuracy.

In addition, four findings of the unclear layer structure A1, the cobblestone-like pattern A2, the cloud-like pattern A3, and the pattern A4 in which liquid accumulation is recognized are given as examples of the findings. However, the findings whose three-dimensional probability maps are generated by the probability map generation unit 10 are not limited to these four findings. For example, probability maps related to five or more findings may be generated.

Further, the configuration in which the presence probabilities of a plurality of findings are calculated using the deep learning method in Step S3 has been described. However, the probability calculation unit 9 can calculate the presence probabilities of a plurality of findings, using a so-called template matching method, a machine learning method using support vector machine (SVM), Adaboost or the like, a machine learning method disclosed in Csurka et al.: Visual Categorization with Bags of Keypoints, Proc. of ECCV Workshop on Statistical Learning in Computer Vision, pp. 59-74 (2004), and the like.

In addition, in Step S5, in a case in which one finding is assigned to each pixel of the ultrasound images of a plurality of frames, a finding having the highest presence probability among the presence probabilities of a plurality of findings included in one pixel is assigned to the pixel. However, a method for assigning the findings to the pixels is not limited thereto. For example, the probability map generation unit 10 has a probability threshold value for the presence probabilities of a plurality of findings and can assign a finding having a presence probability, which is equal to or greater than the probability threshold value and is maximum, among the presence probabilities of a plurality of findings included in one pixel to the pixel. For example, in a case in which the presence probabilities of all of the findings are less than the probability threshold value, the probability map generation unit 10 can determine that the pixels belong to the background region R5 which does not correspond to any findings. As described above, the use of the probability threshold value in a case in which the findings are assigned to each pixel makes it possible to assign more accurate findings to each pixel.

Further, in Step S6, the integrated probability map is displayed on the monitor 6. However, the individual probability map of each finding generated in Step S5 may be displayed. For example, the integrated probability map and the individual probability map of each finding can be automatically displayed together on the monitor 6. In addition, for example, the user can switch the display of the integrated probability map and the individual probability map of each finding through the input device 17 such that the integrated probability map and the individual probability map are displayed separately. This enables the user to more easily and accurately understand the types of a plurality of findings and the presence distribution thereof.

In addition, in a case in which the integrated probability map or the individual probability map of each finding is displayed on the monitor 6 in Step S6, each pixel included in the integrated probability map or the individual probability map of each finding can be displayed in a darker color as the presence probability thereof becomes higher. This enables the user to easily check the presence probability of each finding assigned to a plurality of pixels and to understand the certainty of the assigned finding in the integrated probability map or the individual probability map of each finding.

Further, in a case in which the integrated probability map or the individual probability map of each finding is displayed on the monitor 6 in Step S6, the finding information calculated by the finding information calculation unit 11 can also be displayed. This enables the user to check more detailed information of each finding displayed on the monitor 6 and to more accurately diagnose the decubitus.

Furthermore, in a case in which the integrated probability map or the individual probability map of each finding is displayed on the monitor 6 in Step S6, the failure region detected by the failure region detection unit 12 can also be displayed on the monitor 6. This enables the user to understand that the capture of the ultrasound image has not been performed normally for some reasons, such as the presence of the position where the ultrasound probe 21 is separated from the body surface of the subject, and to perform a re-examination and the like.

Moreover, for example, in a case in which the user checks the integrated probability map or the individual probability map of each finding in Step S6 and determines that a portion different from the portion scanned by the ultrasound probe 21 in Step S2 needs to be additionally scanned, the processes in Steps S1 to S6 can also be performed again in response to an instruction from the user through the input device 17. In this case, in Step S4, a new probability map of each finding is generated and can be combined with, for example, the probability map of each finding that has already been generated and the integrated probability map. This makes it possible to improve the accuracy of the probability map of each finding and the integrated probability map.

### Embodiment 2

In Embodiment 1, the ultrasound images of a plurality of frames are acquired by performing scanning while moving the ultrasound probe 21 in parallel only once in a predetermined direction. However, for example, in a case in which the wound portion J is large and it is not possible to acquire the ultrasound image of a sufficient range with one scanning operation, scanning may be performed while moving the ultrasound probe 21 in parallel a plurality of times in the same direction.

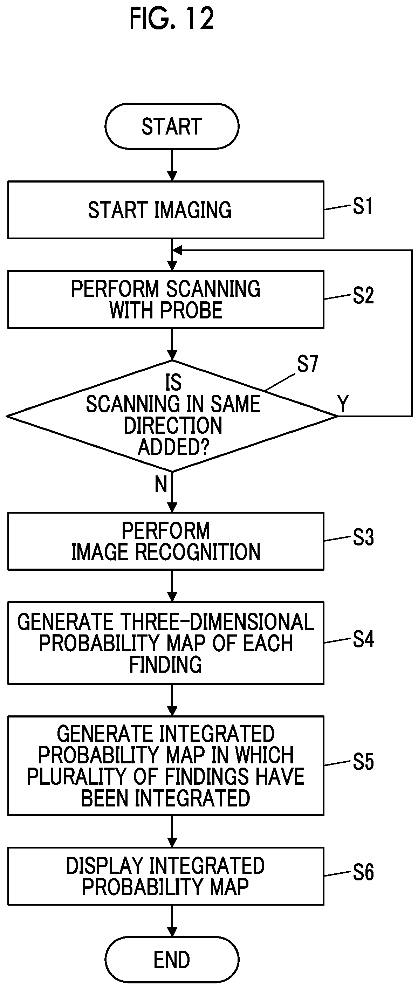

Hereinafter, an operation of an ultrasound diagnostic apparatus 1 according to Embodiment 2 will be described with reference to a flowchart illustrated in Fig. 12. The flowchart illustrated in Fig. 12 is different from the flowchart in Embodiment 1 illustrated in Fig. 10 in that Step S7 is added between Steps S2 and S3.

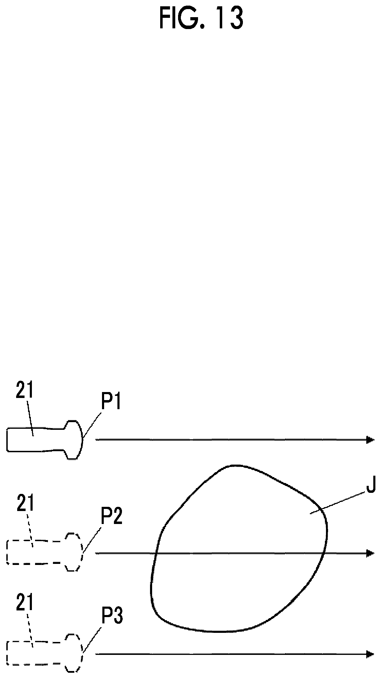

In Step S1, as illustrated in Fig. 13, the capture of ultrasound images is started in a state in which the ultrasound probe 21 is disposed at a position P1 on the body surface of the subject by the user.

Then, in Step S2, the user performs scanning while moving the ultrasound probe 21 in parallel along a predetermined direction to acquire ultrasound images of a plurality of frames. Further, in this case, the position sensor 13 acquires the positional information of the ultrasound probe 21 at any time. In a case in which the ultrasound images of the plurality of frames are acquired in this way, the process proceeds to Step S7.

In Step S7, the device control unit 16 determines whether or not to add scanning in the same direction as the direction in which the ultrasound probe 21 has been moved in parallel in Step S2. For example, in a case in which the user gives an instruction to add scanning in the same direction through the input device 17, the device control unit 16 determines that scanning in the same direction is added, and the process returns to Step S2.

In Step S2, for example, the user disposes the ultrasound probe 21 at a position P2 different from the position P1 and performs scanning with the ultrasound probe 21 while moving the ultrasound probe 21 in parallel in the same direction as that in Step S2 in the previous scanning operation. Therefore, in a case in which the ultrasound images of a plurality of frames are newly acquired and the positional information of the ultrasound probe 21 is newly acquired by the position sensor 13, the process proceeds to Step S7.

In this way, Steps S2 and S7 are repeated until it is determined that scanning in the same direction is not added in Step S7. Then, for example, scanning is performed with the ultrasound probe 21 at a plurality of positions P1 to P3 along the same direction.

For example, in a case in which the user gives an instruction not to add scanning in the same direction through the input device 17 in Step S7, the device control unit 16 determines that scanning in the same direction is not added, and the process proceeds to Step S3.

In Step S3, the probability calculation unit 9 performs image recognition on the ultrasound images of the plurality of frames acquired by repeating Steps S2 and S7 and calculates the presence probability of each finding for each of the ultrasound images of the plurality of frames.

In Step S4, a three-dimensional probability map of each finding is generated on the basis of the positional information of the ultrasound probe 21 acquired by repeating Steps S2 and S7 and the presence probability of each finding calculated in Step S3.

Then, in Step S5, the three-dimensional probability maps of each finding generated in Step S4 are integrated to generate an integrated probability map. In Step S6, the integrated probability map is displayed on the monitor 6.

As described above, according to Embodiment 2, scanning can be performed a plurality of times in the same direction. Therefore, for example, even in a case in which the wound portion J is large and it is not possible to acquire an ultrasound image of a sufficient range with one scanning operation, it is possible to acquire an integrated probability map corresponding to the entire wound portion J and to improve the accuracy of the integrated probability map. In addition, for example, in a case in which scanning is performed with the ultrasound probe 21 at the same position P1 along the same direction, it is possible to improve the accuracy of the integrated probability map even though any of a plurality of scanning operations is not normally performed for some reasons.

### Embodiment 3

In Embodiment 2, scanning is performed with the ultrasound probe 21 a plurality of times along the same direction. However, scanning may be performed with the ultrasound probe 21 along different directions.

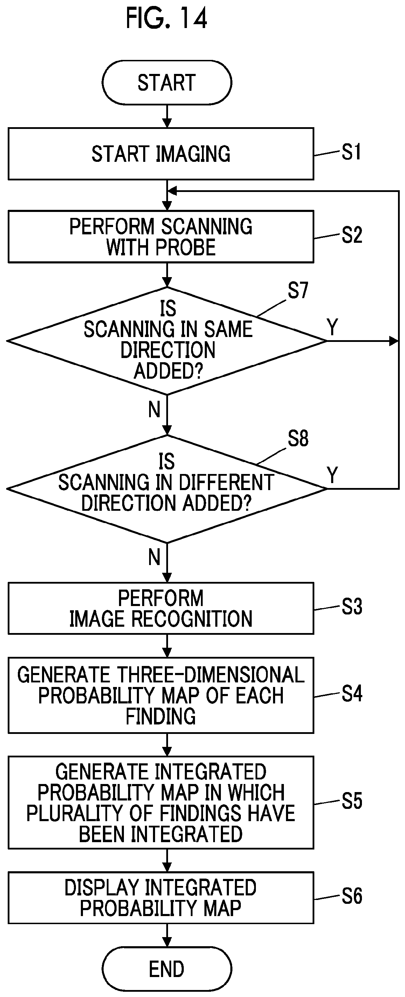

Fig. 14 is a flowchart illustrating an operation of an ultrasound diagnostic apparatus 1 according to Embodiment 3. This flowchart is different from the flowchart in Embodiment 2 illustrated in Fig. 12 in that Step S8 is added between Steps S7 and S3.

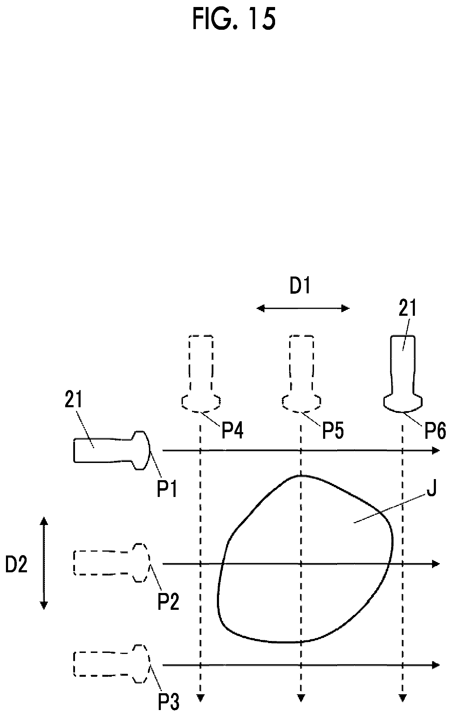

In Step S1, the capture of ultrasound images is started. Then, for example, Step S2 and Step S7 are repeated to perform scanning with the ultrasound probe 21 from each of the positions P1 to P3 along a first direction D1 as illustrated in Fig. 15. Then, in a case in which the device control unit 16 determines that scanning in the same direction as the first direction D1 is not added, the process proceeds to Step S8.

In Step S8, the device control unit 16 determines whether or not to add scanning in a direction different from the first direction D1. For example, in a case in which the user gives an instruction to add scanning in a direction different from the first direction D1 through the input device 17, the device control unit 16 determines that scanning in the direction different from the first direction D1 is added, and the process returns to Step S2.

In Step S2, the user disposes the ultrasound probe 21 at a position P4 different from the positions P1 to P3 and performs scanning with the ultrasound probe 21 while moving the ultrasound probe 21 in parallel along a second direction D2 different from the first direction D1. In this case, the position sensor 13 newly acquires the positional information of the ultrasound probe 21, and the ultrasound images of a plurality of frames are newly acquired.

Then, in Step S7, the device control unit 16 determines whether or not to add scanning in the same direction as the second direction D2 in which the ultrasound probe 21 has been moved in parallel in Step S2 in the previous scanning operation. In a case in which it is determined that scanning in the same direction as the second direction D2 is added, the process returns to Step S2. For example, the ultrasound probe 21 is disposed at a position P5 different from the position P4. Then, scanning is performed with the ultrasound probe 21 while the ultrasound probe 21 is moved in parallel along the second direction D2. Then, in a case in which the positional information of the ultrasound probe 21 and the ultrasound images of a plurality of frames are newly acquired, the process proceeds to Step S7.

For example, Step S2 and Step S7 are repeated in this way to perform scanning with the ultrasound probe 21 from a plurality of positions P4 to P6 along the second direction D2. Then, in a case in which it is determined in Step S7 that scanning in the same direction as the second direction D2 is not added, the process proceeds to Step S8.

In Step S8, it is determined whether or not to add scanning in a direction different from the second direction D2. Here, in a case in which it is determined that scanning in the direction different from the second direction D2 is added, the process returns to Step S2, and scanning in the direction different from the second direction D2 is performed. Further, in a case in which it is determined that scanning in the direction different from the second direction D2 is not added, the process proceeds to Step S3.

In Step S3, image recognition is performed on the ultrasound images of a plurality of frames acquired by repeating Steps S2, S7, and S8, and the presence probability of each finding is calculated for each of the ultrasound images of the plurality of frames.

In Step S4, a three-dimensional probability map of each finding is generated on the basis of the positional information of the ultrasound probe 21 acquired by repeating Steps S2, S7, and S8 and the presence probability of each finding calculated in Step S3.

Then, in Step S5, the three-dimensional probability maps of each finding generated in Step S4 are integrated to generate an integrated probability map. In Step S6, the integrated probability map is displayed on the monitor 6.

As described above, according to Embodiment 3, scanning can be performed a plurality of times in a plurality of directions. Therefore, for example, even in a case in which the wound portion J is large and it is not possible to acquire an ultrasound image of a sufficient range with one scanning operation, it is possible to acquire an integrated probability map corresponding to the entire wound portion J and to improve the accuracy of the integrated probability map.

Further, in a case in which scanning is performed with the ultrasound probe 21 along a plurality of directions, some regions are scanned to overlap each other. In the overlapping scanned region, the presence probability of each finding is calculated a plurality of times. Therefore, it is considered that the reliability of the presence probability of each finding is higher than that of other regions. Therefore, for example, the probability map generation unit 10 can generate the probability map of each finding and the integrated probability map in which the overlapping scanned region has a darker color such that the user can easily understand the reliability of the presence probability of each finding. In addition, the probability map generation unit 10 can also generate the probability map of each finding and the integrated probability map in which the overlapping scanned region has higher density.

### Embodiment 4

In Embodiment 2 and Embodiment 3, scanning is performed with the ultrasound probe 21 a plurality of times along the same direction or different directions. However, in a case in which the region that is currently being scanned by the ultrasound probe 21 has already been scanned, it is possible to notify the user of the fact.

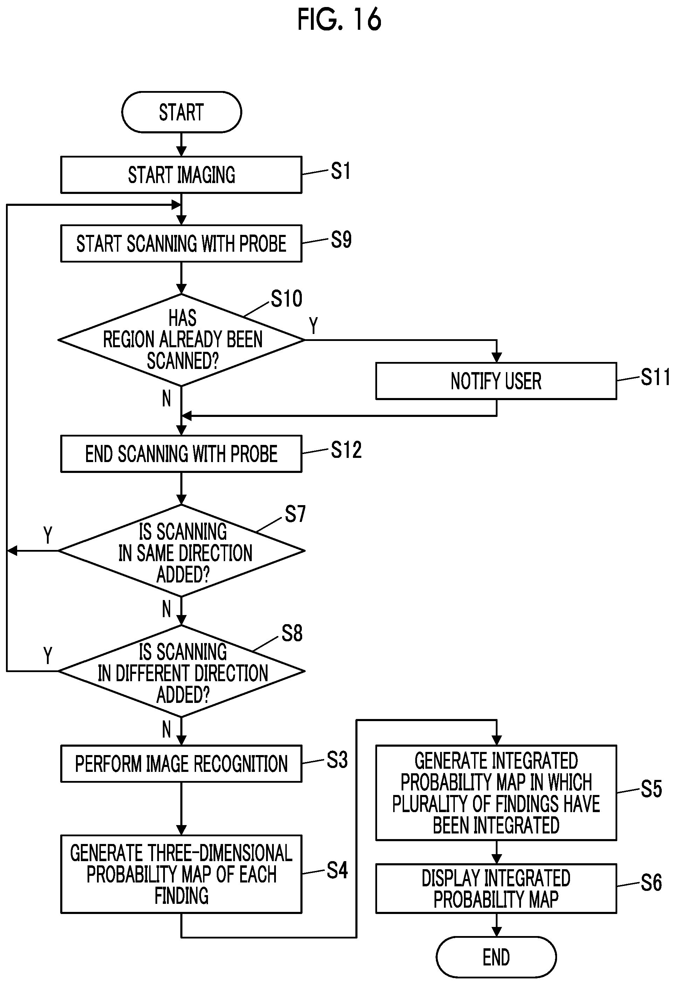

Hereinafter, an operation of an ultrasound diagnostic apparatus 1 according to Embodiment 4 will be described with reference to a flowchart illustrated in Fig. 16. This flowchart is different from the flowchart in Embodiment 3 illustrated in Fig. 14 in that Steps S9 to S12 are added instead of Step S2.

First, in a case in which the capture of ultrasound images is started in Step S1, the process proceeds to Step S9.

In Step S9, scanning with the ultrasound probe 21 is started. Then, the user starts scanning while moving the ultrasound probe 21 in parallel along a predetermined direction. In this case, the position sensor 13 acquires the positional information of the ultrasound probe 21, and the acquired positional information is stored in the image memory 8 to be associated with the ultrasound image of the frame acquired at the same timing.

In Step S10, the scanned region detection unit 14 performs a process of detecting whether or not the region that is currently being scanned by the ultrasound probe 21 is the region that has already been scanned, on the basis of the positional information of the ultrasound probe 21 acquired by the position sensor 13. For example, the scanned region detection unit 14 collates the positional information of the ultrasound probe 21 which has already been acquired in the same examination with the newly acquired positional information. In a case in which the positional information items are matched with each other, it is possible to detect that the region that is currently being scanned by the ultrasound probe 21 is the region that has already been scanned.

Since the current operation is the first scanning operation, it is determined that the region that is currently being scanned by the ultrasound probe 21 is not the region that has already been scanned, and the process proceeds to Step S12.

In Step S12, for example, in a case in which the user gives an instruction to end the scanning with the ultrasound probe 21 through the input device 17, the scanning with the ultrasound probe 21 ends.

Then, in Step S7, the device control unit 16 determines whether or not to add scanning in the same direction as the direction in which the ultrasound probe 21 has been moved in parallel between Step S9 and Step S12. Here, in a case in which it is determined that scanning in the same direction is added, the process returns to Step S9.

In Step S9, scanning with the ultrasound probe 21 is started while the ultrasound probe 21 is moved in the same direction as the direction in which the ultrasound probe 21 has been moved in parallel in Step S9 in the first scanning operation.

Then, in Step S10, the positional information of the ultrasound probe 21 acquired in the first scanning operation is collated with the positional information of the ultrasound probe 21 newly acquired in the current scanning operation. In a case in which the positional information items are matched with each other, it is determined that the region that is currently being scanned by the ultrasound probe 21 is the region that has already been scanned, and the process proceeds to Step S11.

In Step S11, the notification unit 15 notifies the user that the region that is currently being scanned by the ultrasound probe 21 is the region that has already been scanned. The notification unit 15 can display a message on the monitor 6 to notify the user, which is not illustrated. In a case in which the process in Step S11 is completed in this way, the process proceeds to Step S12, and the scanning with the ultrasound probe 21 ends.

Since the subsequent Steps S7 and S8 and Steps S3 to S6 are the same as Steps S3 to S6 in Embodiments 1 to 3, the description thereof will not be repeated.

As described above, in Embodiment 4, it is detected whether or not the region that is currently being scanned by the ultrasound probe 21 is the region that has already been scanned. In a case in which the region that is currently being scanned by the ultrasound probe 21 is the region that has already been scanned, the user is notified of the fact. Therefore, for example, the user can be prevented from unintentionally scanning the same portion.

Further, in Step S11, the notification unit 15 displays a message on the monitor 6 to notify the user. However, a method for notifying the user is not limited thereto. For example, in a case in which the ultrasound diagnostic apparatus 1 comprises a speaker, the notification unit 15 can emit a voice from the speaker to notify the user. In addition, for example, in a case in which the ultrasound diagnostic apparatus 1 comprises a lamp, the notification unit 15 can emit light from the lamp to notify the user. Further, for example, in a case in which a motor or the like for vibrating the ultrasound probe 21 is attached to the ultrasound probe 21, the notification unit 15 can vibrate the ultrasound probe 21 to notify the user.

### Embodiment 5

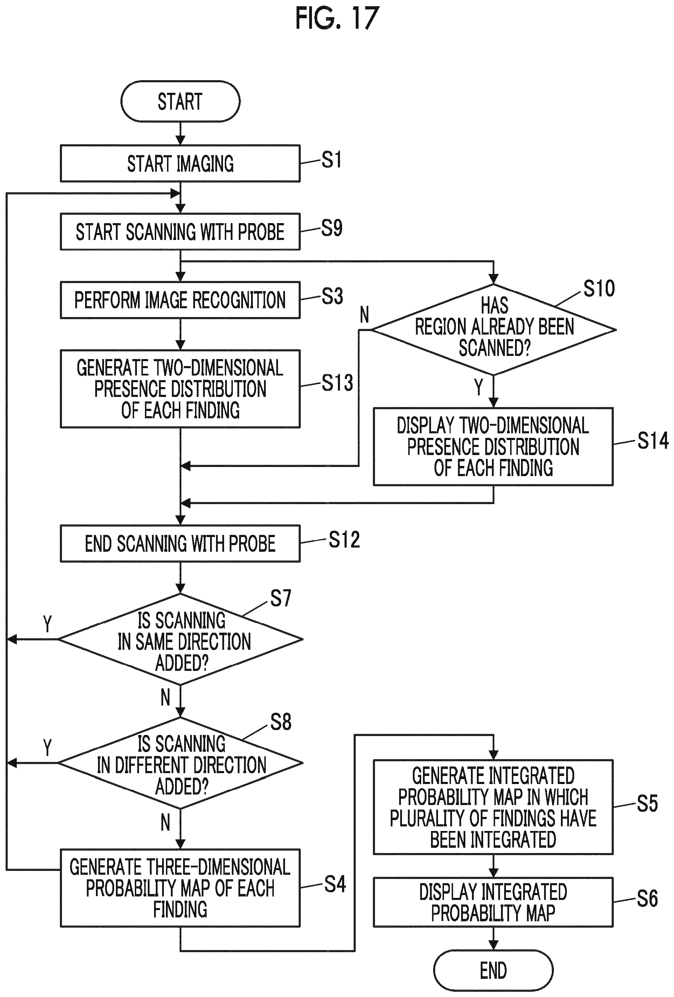

In Embodiment 4, for example, the notification unit 15 displays a message on the monitor 6 to notify the user. However, the notification unit 15 may be connected to the probability map generation unit 10 and may display the probability map generated in the region, which has already been scanned, on the monitor 6 to notify the user. In this case, the ultrasound diagnostic apparatus 1 can operate according to, for example, a flowchart illustrated in Fig. 17.

The flowchart illustrated in Fig. 17 is different from the flowchart illustrated in Fig. 16 in that Step S3, Step S10, Step S13, and Step S14 are processed between Steps S9 and S12, instead of Step S10 and Step S11.

In a case in which scanning with the ultrasound probe 21 is started in Step S9, Steps S3 and S10 are processed in parallel.

In Step S3, whenever scanning is performed with the ultrasound probe 21 to acquire an ultrasound image, the probability calculation unit 9 performs image recognition on the acquired ultrasound image of the frame to calculate the presence probability of each finding at any time.

Then, in Step S13, a two-dimensional presence distribution of each finding is generated for each ultrasound image on the basis of the presence probability of each finding calculated in Step S3.

In addition, in Step S10, the scanned region detection unit 14 performs a process of detecting whether or not the region that is currently being scanned by the ultrasound probe 21 is the region that has already been scanned. Since the current operation is the first scanning operation, it is determined that the region that is currently being scanned by the ultrasound probe 21 is not the region that has already been scanned.

Then, in Step S13, the two-dimensional presence distribution of each finding is generated. In a case in which it is determined in Step S10 that the region that is currently being scanned by the ultrasound probe 21 is not the region that has already been scanned, the process proceeds to Step S12.

In Step S12, the scanning with the ultrasound probe 21 ends. Then, the process proceeds to Step S7.

In Step S7, it is determined whether or not scanning in the same direction as the direction in which the ultrasound probe 21 has been moved in parallel between Steps S9 and S12 is added. In a case in which it is determined that scanning in the same direction is added, the process returns to Step S9.

In Step S9, in a case in which scanning with the ultrasound probe 21 is started again, Steps S3 and S13 are processed as in the first scanning operation.

In addition, in Step S10, it is determined whether or not the region that is currently being scanned by the ultrasound probe 21 is the region that has already been scanned in the first scanning operation. In a case in which it is determined that the region that is currently being scanned by the ultrasound probe 21 is the region that has already been scanned in the first scanning operation, the process proceeds to Step S14.

In Step S14, the notification unit 15 notifies the user by displaying the two-dimensional presence distribution of each finding generated in Step S13 in the first scanning operation which corresponds to the region determined in Step S10 on the monitor 6 to be superimposed on the ultrasound image of the corresponding frame.

Therefore, the user can understand that the region that is currently being scanned by the ultrasound probe 21 is the region that has already been scanned. As a result, for example, it is possible to prevent the unintentional scanning of the same portion.

As described above, in a case in which the processes in Step S13 and Step S14 are completed, the process proceeds to Step S12. Since the subsequent Steps S12, S7, S8, and S4 to S6 are the same as those described in the flowchart illustrated in Fig. 16, the description thereof will not be repeated.

As described above, according to Embodiment 5, even in a case in which the image recognition in Step S3 and the determination of whether or not the region being scanned by the ultrasound probe 21 is the region that has already been scanned in Step S10 are performed in parallel, for example, the user can be prevented from unintentionally scanning the same portion as in Embodiment 4.

### Embodiment 6

In Embodiments 1 to 5, the three-dimensional probability map of each finding is generated after the acquisition of all of the ultrasound images ends. However, the probability map of each finding may be generated whenever one scanning operation ends.

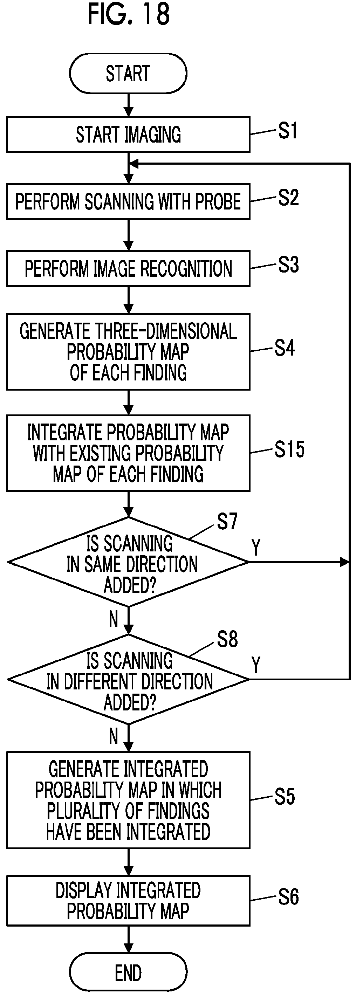

Fig. 18 is a flowchart illustrating an operation of an ultrasound diagnostic apparatus 1 according to Embodiment 6. This flowchart is different from the flowchart in Embodiment 3 illustrated in Fig. 14 in that the positions of Step S3 and Step S4 are moved between Steps S2 and S7 and Step S15 is added immediately after Step S4.

In a case in which the capture of ultrasound images is started in Step S1, the process proceeds to Step S2.

In Step S2, for example, as illustrated in Fig. 15, in a state in which the ultrasound probe 21 is disposed at the position P1, the user performs scanning with the ultrasound probe 21 while moving the ultrasound probe 21 in parallel along the first direction D1 to acquire ultrasound images of a plurality of frames. Further, in this case, the position sensor 13 acquires the positional information of the ultrasound probe 21 at any time.

Then, in Step S3, the probability calculation unit 9 performs image recognition on the ultrasound images of the plurality of frames acquired in Step S2 and calculates the presence probability of each finding for each ultrasound image of each frame.

In Step S4, the probability map generation unit 10 generates a three-dimensional probability map of each finding on the basis of the positional information of the ultrasound probe 21 acquired in Step S2 and the presence probability of each finding calculated in Step S3.

Then, in Step S15, in a case in which the three-dimensional probability map of each finding has already been generated before the previous Step S4, the probability map generation unit 10 performs a process of integrating the probability map generated in the previous Step S4 with the existing probability map for each finding. At present, only the first scanning operation is performed, and the existing probability map is not present. Therefore, the probability map of each finding generated in the previous Step S4 is not integrated with the other probability maps.

In a case in which the process in Step S15 is completed in this way, the process proceeds to Step S7.

In Step S7, it is determined whether or not scanning with the ultrasound probe 21 is performed along the same direction as the first direction D1. In a case in which it is determined that scanning in the same direction is added, the process returns to Step S2.

In Step S2, for example, scanning with the ultrasound probe 21 is performed along the first direction D1 at the position P2 different from the position P1 in Step S2 in the first scanning operation. Therefore, the ultrasound images of a plurality of frames are newly acquired, and the positional information of the ultrasound probe 21 is also newly acquired.

Then, in Step S3, image recognition is performed on the ultrasound images of the plurality of frames obtained in Step S2 in the second scanning operation, and the presence probability of each finding is calculated.

Further, in Step S4, a three-dimensional probability map of each finding is newly generated on the basis of the positional information of the ultrasound probe 21 acquired in Step S2 in the second scanning operation and the presence probability of each finding obtained in Step S3 in the second scanning operation.

Then, in Step S15, the probability map generation unit 10 integrates the probability map of each finding generated in Step S4 in the first scanning operation and the probability map of each finding generated in Step S4 in the second scanning operation for each finding. Therefore, the probability map of each finding with higher accuracy than the probability map of each finding generated in Step S4 in the first scanning operation is obtained.

Steps S2 to S4, Step S15, and Step S7 are repeated in this way to integrate the probability maps obtained by a plurality of scanning operations along the first direction D1 for each finding. In a case in which it is determined in Step S7 that scanning in the same direction as the first direction D1 is not added, the process proceeds to Step S8.

In Step S8, it is determined whether or not to add scanning in a direction different from the first direction D1. Here, in a case in which it is determined that scanning in the direction different from the first direction D1 is added, the process returns to Step S2.

In Step S2, for example, as illustrated in Fig. 15, in a state in which the ultrasound probe 21 is disposed at the position P4, the user performs scanning with the ultrasound probe 21 along the second direction D2 different from the first direction D1. Therefore, the ultrasound images of a plurality of frames are newly acquired, and the positional information of the ultrasound probe 21 is newly acquired.

Then, in Step S3, image recognition is performed on the ultrasound images of the plurality of frames newly acquired in Step S2 to calculate the presence probability of each finding.

In Step S4, the three-dimensional presence probability of each finding is newly generated on the basis of the positional information of the ultrasound probe 21 newly acquired in Step S2 and the presence probability of each finding newly calculated in Step S3.

Then, in Step S15, for each finding, the probability map newly generated in Step S4 is integrated with the existing probability map obtained as a result of the scanning with the ultrasound probe 21 along the first direction D1. In a case in which the process in Step S15 is completed in this way, the process proceeds to Step S7.

In Step S7, it is determined whether or not to add scanning in the same direction as the second direction D2. In a case in which it is determined to add scanning in the same direction as the second direction D2, the process returns to Step S2. In a case in which it is determined not to add scanning in the same direction as the second direction D2, the process proceeds to Step S8.

Steps S2 to S4, Step S15, Step S7, and Step S8 are repeated in this way to integrate the newly generated probability map with the existing probability map for each finding.

In a case in which it is determined not to add scanning in a different direction in Step S8, the process proceeds to Step S5.

In Step S5, the three-dimensional probability maps of each finding obtained by repeating Steps S2 to S4, Step S15, Step S7, and Step S8 are integrated to generate an integrated probability map.

Then, in Step S6, the integrated probability map generated in Step S5 is displayed on the monitor 6.

Then, the operation of the ultrasound diagnostic apparatus 1 according to Embodiment 6 ends.

As described above, even in a case in which the three-dimensional probability map of each finding is generated for each scanning operation with the ultrasound probe 21, the three-dimensional probability maps of a plurality of findings are integrated to generate the integrated probability map, and the generated integrated probability map is displayed on the monitor 6. Therefore, the user can easily and accurately understand the type of finding of a decubitus and the three-dimensional distribution thereof, as in Embodiment 1.

### Embodiment 7

The ultrasound diagnostic apparatus 1 according to Embodiment 1 has the configuration in which the monitor 6, the input device 17, and the ultrasound probe 21 are directly connected to the processor 22. However, for example, the monitor 6, the input device 17, and the ultrasound probe 21, and the processor 22 can also be indirectly connected to each other through a network.

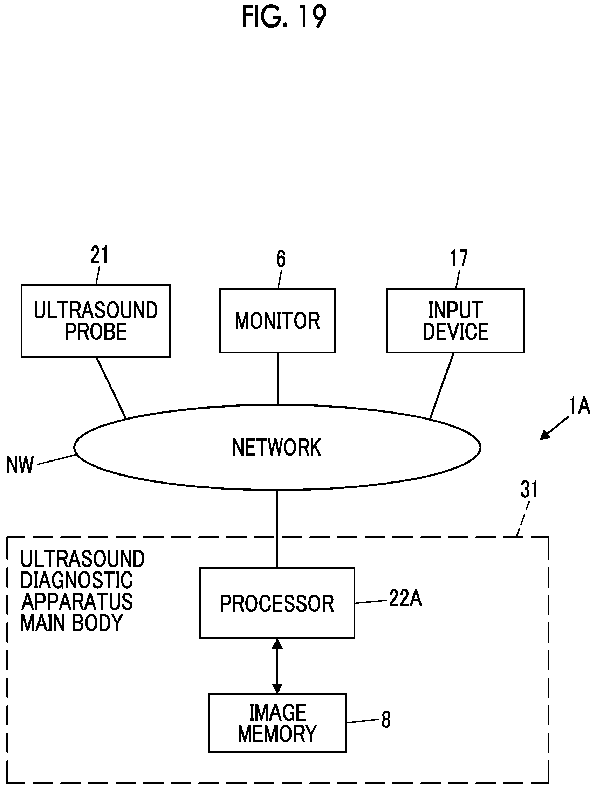

As illustrated in Fig. 19, in an ultrasound diagnostic apparatus 1A according to Embodiment 7, the monitor 6, the input device 17, and the ultrasound probe 21 are connected to an ultrasound diagnostic apparatus main body 31 through a network NW. The ultrasound diagnostic apparatus main body 31 is composed of the image memory 8 and a processor 22A, excluding the monitor 6, the input device 17, and the ultrasound probe 21 in the ultrasound diagnostic apparatus 1 according to Embodiment 1 illustrated in Fig. 1.

Even in a case in which the ultrasound diagnostic apparatus 1A has this configuration, as in the ultrasound diagnostic apparatus 1 according to Embodiment 1, image recognition is performed on the acquired ultrasound images of a plurality of frames to calculate the presence probability of each finding, a three-dimensional probability map of each finding is generated on the basis of the presence probability of each finding and the positional information of the ultrasound probe 21 acquired by the position sensor 13, the probability maps of a plurality of findings are integrated to generate a three-dimensional integrated probability map, and the integrated probability map is displayed on the monitor 6. Therefore, according to the ultrasound diagnostic apparatus 1A of Embodiment 7, as in the ultrasound diagnostic apparatus 1 of Embodiment 1, the user can easily and accurately understand the type of finding of a decubitus and the three-dimensional distribution thereof.

Further, since the monitor 6, the input device 17, and the ultrasound probe 21 are connected to the ultrasound diagnostic apparatus main body 31 through a network NW, the ultrasound diagnostic apparatus main body 31 can be used as a so-called remote server. Therefore, for example, the user can prepare the monitor 6, the input device 17, and the ultrasound probe 21 at hand and diagnose the subject. Therefore, it is possible to improve convenience in ultrasound diagnosis.

In addition, the monitor 6, the input device 17, and the ultrasound probe 21 are connected to the ultrasound diagnostic apparatus main body 31 through the network NW. However, in this case, the monitor 6, the input device 17, and the ultrasound probe 21 may be connected to the network NW in a wired manner or wirelessly.

Further, the application of the aspect of Embodiment 7 to Embodiment 1 has been described. However, the aspect of Embodiment 7 is also applied to Embodiments 2 to 6.

### Explanation of References

1, 1A: ultrasound diagnostic apparatus
2: transducer array
3: transmitting and receiving circuit
4: image generation unit
5: display control unit
6: monitor
7: image acquisition unit
8: image memory
9: probability calculation unit
10: probability map generation unit
11: finding information calculation unit
12: failure region detection unit
13: position sensor
14: scanned region detection unit
15: notification unit
16: device control unit
17: input device
21: ultrasound probe
22, 22A: processor
23: pulser
24: amplification unit
25: AD conversion unit
26: beam former
27: signal processing unit
28: DSC
29: image processing unit
31: ultrasound diagnostic apparatus main body
A1: unclear layer structure
A2: cobblestone-like pattern
A3: cloud-like pattern
A4: pattern
B1: scanning cross section
D1: first direction
D2: second direction
F: hollow region
J: wound portion
M: probability map
NW: network
P1 to P6: position
R1 to R4, T: region
R5: background region

## Claims

1. An ultrasound diagnostic apparatus (1A) comprising:
an ultrasound probe (21);
a position sensor (13) that is attached to the ultrasound probe (21) and acquires positional information of the ultrasound probe (21);
an image acquisition unit (7) that scans a wound portion (J) of a subject with an ultrasound beam using the ultrasound probe (21) to acquire ultrasound images of a plurality of frames;
a probability calculation unit (9) that calculates a presence probability of a finding related to the wound portion (J) from each of the ultrasound images of the plurality of frames, wherein said finding is a structure and pattern of tissues of the subject corresponding to decubitus symptoms such as edema, necrosis and abscess; and
a probability map generation unit (10) that generates a three-dimensional probability map (M) of the finding on the basis of the positional information of the ultrasound probe (21) acquired by the position sensor (13) and the presence probability calculated by the probability calculation unit (9).

2. The ultrasound diagnostic apparatus (1A) according to claim 1,
wherein the probability calculation unit (9) calculates a plurality of the presence probabilities corresponding to each of a plurality of predetermined findings, and
the probability map generation unit (10) three-dimensionally visualizes each of the plurality of presence probabilities and generates a three-dimensional probability map (M), in which the plurality of findings have been integrated, on the basis of the visualized plurality of presence probabilities.

3. The ultrasound diagnostic apparatus (1A) according to claim 1 or 2, further comprising:
a monitor (6) that displays the probability map.

4. The ultrasound diagnostic apparatus according to claim 3, further comprising:
a finding information calculation unit (11) that calculates finding information including at least one of a size, a depth, or a volume of the finding on the basis of the probability map (M), and
wherein the monitor (6) displays the finding information.

5. The ultrasound diagnostic apparatus (1A) according to claim 3 or 4,
wherein the probability map generation unit (10) generates the probability map (M) in which a shade of a color is changed according to a value of the presence probability calculated by the probability calculation unit (9).

6. The ultrasound diagnostic apparatus (1A) according to any one of claims 3 to 5,
wherein, in a case in which a plurality of scanning operations are performed on the same wound portion (J), the probability map generation unit (10) generates the probability map on the basis of a plurality of the presence probabilities calculated by the probability calculation unit (9) corresponding to the plurality of scanning operations.

7. The ultrasound diagnostic apparatus (1A) according to any one of claims 3 to 5,
wherein, in a case in which a plurality of scanning operations are performed on the same wound portion (J), the probability map generation unit (10) generates the probability map for each of the scanning operations, integrates a plurality of the probability maps generated corresponding to the plurality of scanning operations, and displays an integrated probability map on the monitor (6).

8. The ultrasound diagnostic apparatus (1A) according to any one of claims 3 to 7, further comprising:
a failure region detection unit (12) that analyzes the probability map or the ultrasound image to detect a failure region in which the probability map (M) is not normally generated or the ultrasound image is not normally acquired,
wherein the monitor (6) displays the failure region.

9. The ultrasound diagnostic apparatus (1A) according to any one of claims 3 to 8, further comprising:
a scanned region detection unit (14) that detects that a region (R1 to R4, T) has already been scanned on the basis of the positional information of the ultrasound probe (21) acquired by the position sensor in a case in which scanning is performed on the wound portion; and
a notification unit (15) that notifies a user in a case in which it is detected that the region has already been scanned.

10. The ultrasound diagnostic apparatus (1A) according to any one of claims 3 to 9,
wherein the monitor (6) displays the probability map that has already been generated by the probability map generation unit (10) on the basis of the positional information of the ultrasound probe (21) acquired by the position sensor in a case in which scanning is performed on the wound portion (J).

11. The ultrasound diagnostic apparatus (1A) according to any one of claims 3 to 10,
wherein the probability map generation unit (10) generates the probability map in which a region, which has been repeatedly scanned a larger number of times, has a darker color or a higher density.

12. A method for controlling an ultrasound diagnostic apparatus (1A), the method comprising:
acquiring positional information of an ultrasound probe (21);
scanning a wound portion (J) of a subject with an ultrasound beam using the ultrasound probe (21) to acquire ultrasound images of a plurality of frames;
calculating a presence probability of a finding related to the wound portion (J) from each of the ultrasound images of the plurality of frames, wherein said finding is a structure and pattern of tissues of the subject corresponding to decubitus symptoms such as edema, necrosis and abscess; and
generating a three-dimensional probability map (M) of the finding on the basis of the acquired positional information of the ultrasound probe (21) and the calculated presence probability.

13. A processor (22, 22A) for an ultrasound diagnostic apparatus (1A), the processor (22, 22A) being configured to:
acquire positional information of an ultrasound probe (21);
scan a wound portion (J) of a subject with an ultrasound beam using the ultrasound probe (21) to acquire ultrasound images of a plurality of frames;
calculate a presence probability of a finding related to the wound portion (J) from each of the ultrasound images of the plurality of frames, wherein said finding is a structure and pattern of tissues of the subject corresponding to decubitus symptoms such as edema, necrosis and abscess; and
generate a three-dimensional probability map (M) of the finding on the basis of the acquired positional information of the ultrasound probe (21) and the calculated presence probability.

## Patentansprüche

1. Ultraschalldiagnosevorrichtung (1A), umfassend:
eine Ultraschallsonde (21);
einen Positionssensor (13), der an der Ultraschallsonde (21) angebracht ist und
Positionsinformationen der Ultraschallsonde (21) erfasst;
eine Bilderfassungseinheit (7), die einen Wundabschnitt (J) einer Untersuchungsperson mit einem Ultraschallstrahl unter Verwendung der Ultraschallsonde (21) abtastet, um Ultraschallbilder von mehreren Einzelbildern zu erfassen;
eine Wahrscheinlichkeitsberechnungseinheit (9), die eine
Vorhandenseinswahrscheinlichkeit einer Befundes, der sich auf den Wundabschnitt (J) bezieht, aus jedem der Ultraschallbilder der mehreren Einzelbilder berechnet, wobei der Befund eine Struktur und ein Muster von Geweben der Untersuchungsperson ist, die Decubitus-Symptomen, wie beispielsweise Ödem, Nekrose und Abszess, entsprechen; und
eine Wahrscheinlichkeitskarten-Erzeugungseinheit (10), die eine dreidimensionale Wahrscheinlichkeitskarte (M) des Befunds auf der Grundlage der Positionsinformationen der Ultraschallsonde (21), die von dem Positionssensor (13) erfasst worden sind, und der von der Wahrscheinlichkeitsberechnungseinheit (9) berechneten Vorhandenseinswahrscheinlichkeit erzeugt.

2. Ultraschalldiagnosevorrichtung (1A) nach Anspruch 1,
wobei die Wahrscheinlichkeitsberechnungseinheit (9) mehrere der Vorhandenseinswahrscheinlichkeiten, die jedem von mehreren vorbestimmten Befunden entsprechen, berechnet, und
die Wahrscheinlichkeitskarten-Erzeugungseinheit (10) jede der mehreren Vorhandenseinswahrscheinlichkeiten dreidimensional visualisiert und eine dreidimensionale Wahrscheinlichkeitskarte (M), in der die mehreren Befunde integriert worden sind, auf der Grundlage der visualisierten mehreren Vorhandenseinswahrscheinlichkeiten erzeugt.

3. Ultraschalldiagnosevorrichtung (1A) nach Anspruch 1 oder 2, ferner umfassend:
einen Monitor (6), der die Wahrscheinlichkeitskarte anzeigt.

4. Ultraschalldiagnosevorrichtung nach Anspruch 3, ferner umfassend:
eine Befundinformations-Berechnungseinheit (11), die Befundinformationen, die mindestens eine von einer Größe, einer Tiefe und einem Volumen des Befunds enthalten, auf der Grundlage der Wahrscheinlichkeitskarte (M) berechnet, und
wobei der Monitor (6) die Befundinformationen anzeigt.

5. Ultraschalldiagnosevorrichtung (1A) nach Anspruch 3 oder 4,
wobei die Wahrscheinlichkeitskarten-Erzeugungseinheit (10) die Wahrscheinlichkeitskarte (M) erzeugt, bei der ein Farbton gemäß einem Wert der von der Wahrscheinlichkeitsberechnungseinheit (9) berechneten Vorhandenseinswahrscheinlichkeit geändert wird.

6. Ultraschalldiagnosevorrichtung (1A) nach einem der Ansprüche 3 bis 5,
wobei in einem Fall, in dem mehrere Abtastvorgänge an demselben Wundabschnitt (J) durchgeführt werden, die Wahrscheinlichkeitskarten-Erzeugungseinheit (10) die Wahrscheinlichkeitskarte auf der Grundlage mehrerer der von der Wahrscheinlichkeitsberechnungseinheit (9) berechneten Vorhandenseinswahrscheinlichkeiten, die den mehreren Abtastvorgängen entsprechen, erzeugt.

7. Ultraschalldiagnosevorrichtung (1A) nach einem der Ansprüche 3 bis 5,
wobei in einem Fall, in dem mehrere Abtastvorgänge an demselben Wundabschnitt (J) durchgeführt werden, die Wahrscheinlichkeitskarten-Erzeugungseinheit (10) die Wahrscheinlichkeitskarte für jeden der Abtastvorgänge erzeugt, mehrere der Wahrscheinlichkeitskarten, die entsprechend den mehreren Abtastvorgängen erzeugt worden sind, integriert und eine integrierte Wahrscheinlichkeitskarte auf dem Monitor (6) anzeigt.

8. Ultraschalldiagnosevorrichtung (1A) nach einem der Ansprüche 3 bis 7, ferner umfassend:
eine Ausfallbereichs-Detektionseinheit (12), die die Wahrscheinlichkeitskarte oder das Ultraschallbild analysiert, um einen Ausfallbereich, in dem die Wahrscheinlichkeitskarte (M) nicht normal erzeugt wird oder das Ultraschallbild nicht normal erfasst wird, zu detektieren,
wobei der Monitor (6) den Ausfallbereich anzeigt.

9. Ultraschalldiagnosevorrichtung (1A) nach einem der Ansprüche 3 bis 8, ferner umfassend:
eine Abtastbereichs-Detektionseinheit (14), die detektiert, dass ein Bereich (R1 bis R4, T) bereits abgetastet worden ist, auf der Grundlage der Positionsinformationen der Ultraschallsonde (21), die von dem Positionssensor in einem Fall, in dem Abtasten an dem Wundabschnitt durchgeführt wird, erfasst worden sind; und
eine Benachrichtigungseinheit (15), die einen Benutzer in einem Fall benachrichtigt, in dem detektiert wird, dass der Bereich bereits abgetastet worden ist.

10. Ultraschalldiagnosevorrichtung (1A) nach einem der Ansprüche 3 bis 9,
wobei der Monitor (6) die Wahrscheinlichkeitskarte, die bereits von der Wahrscheinlichkeitskarten-Erzeugungseinheit (10) erzeugt worden ist, auf der Grundlage der Positionsinformationen der Ultraschallsonde (21), die von dem Positionssensor in einem Fall, in dem Abtasten an dem Wundabschnitt (J) durchgeführt wird, erfasst worden ist, anzeigt.

11. Ultraschalldiagnosevorrichtung (1A) nach einem der Ansprüche 3 bis 10,
wobei die Wahrscheinlichkeitskarten-Erzeugungseinheit (10) die Wahrscheinlichkeitskarte erzeugt, bei der ein Bereich, der wiederholt eine größere Anzahl von Malen abgetastet worden ist, eine dunklere Farbe oder eine höhere Dichte aufweist.

12. Verfahren zum Steuern einer Ultraschalldiagnosevorrichtung (1A), wobei das Verfahren umfasst:
Erfassen von Positionsinformationen einer Ultraschallsonde (21);
Abtasten eines Wundabschnitts (J) einer Untersuchungsperson mit einem Ultraschallstrahl unter Verwendung der Ultraschallsonde (21), um Ultraschallbilder von mehreren Einzelbildern zu erfassen;
Berechnen einer Vorhandenseinswahrscheinlichkeit eines Befunds, der sich auf den Wundabschnitt (J) bezieht, aus jedem der Ultraschallbilder der mehreren Einzelbilder,
wobei der Befund eine Struktur und ein Muster von Geweben der Untersuchungsperson ist, die Decubitus-Symptomen, wie beispielsweise Ödem, Nekrose und Abszess, entsprechen; und
Erzeugen einer dreidimensionalen Wahrscheinlichkeitskarte (M) des Befunds auf der Grundlage der erfassten Positionsinformationen der Ultraschallsonde (21) und der berechneten Vorhandenseinswahrscheinlichkeit.

13. Prozessor (22, 22A) für eine Ultraschalldiagnosevorrichtung (1A), wobei der Prozessor (22, 22A) so konfiguriert ist, dass er:
Positionsinformationen einer Ultraschallsonde (21) erfasst;
einen Wundabschnitt (J) einer Untersuchungsperson mit einem Ultraschallstrahl unter Verwendung der Ultraschallsonde (21) abtastet, um Ultraschallbilder von mehreren Einzelbildern zu erfassen;
eine Vorhandenseinswahrscheinlichkeit eines Befunds, der sich auf den Wundabschnitt (J) bezieht, aus jedem der Ultraschallbilder der mehreren Einzelbilder berechnet, wobei der Befund eine Struktur und ein Muster von Geweben der Untersuchungsperson ist, die Decubitus-Symptomen, wie beispielsweise Ödem, Nekrose und Abszess, entsprechen; und
eine dreidimensionale Wahrscheinlichkeitskarte (M) des Befunds auf der Grundlage der erfassten Positionsinformationen der Ultraschallsonde (21) und der berechneten Vorhandenseinswahrscheinlichkeit erzeugt.

## Revendications

1. Appareil de diagnostic par ultrasons (1A) comprenant :
une sonde ultrasonore (21) ;
un capteur de position (13) qui est fixé à la sonde ultrasonore (21) et acquiert des informations de position de la sonde ultrasonore (21) ;
une unité d'acquisition d'images (7) qui balaye une partie de plaie (J) d'un sujet avec un faisceau ultrasonore en utilisant la sonde ultrasonore (21) pour acquérir des images ultrasonores d'une pluralité de trames ;
une unité de calcul de probabilité (9) qui calcule une probabilité de présence d'une anomalie liée à la partie de plaie (J) à partir de chacune des images ultrasonores de la pluralité de trames, dans laquelle ladite anomalie est une structure et un schéma de tissus du sujet correspondant à des symptômes de décubitus tels que l'œdème, la nécrose et l'abcès ; et
une unité de génération de cartes de probabilité (10) qui génère une carte de probabilité tridimensionnelle (M) de l'anomalie sur la base des informations de position de la sonde ultrasonore (21) acquises par le capteur de position (13) et de la probabilité de présence calculée par l'unité de calcul de probabilité (9).

2. Appareil de diagnostic par ultrasons (1A) selon la revendication 1,
dans lequel l'unité de calcul de probabilité (9) calcule une pluralité de probabilités de présence correspondant à chacune d'une pluralité d'anomalies prédéterminées, et l'unité de génération de cartes de probabilité (10) visualise en trois dimensions chacune de la pluralité de probabilités de présence et génère une carte de probabilité tridimensionnelle (M), dans laquelle la pluralité d'anomalies a été intégrée, sur la base de la pluralité de probabilités de présence visualisées.

3. Appareil de diagnostic par ultrasons (1A) selon la revendication 1 ou la revendication 2, comprenant en outre :
un moniteur (6) qui affiche la carte de probabilité.

4. Appareil de diagnostic par ultrasons selon la revendication 3, comprenant en outre :
une unité de calcul d'informations d'anomalie (11) qui calcule des informations d'anomalie incluant au moins l'une d'une taille, d'une profondeur ou d'un volume de l'anomalie sur la base de la carte de probabilité (M), et
dans lequel le moniteur (6) affiche les informations d'anomalie.

5. Appareil de diagnostic par ultrasons (1A) selon la revendication 3 ou la revendication 4,
dans lequel l'unité de génération de cartes de probabilité (10) génère la carte de probabilité (M) dans laquelle une teinte d'une couleur est modifiée en fonction d'une valeur de la probabilité de présence calculée par l'unité de calcul de probabilité (9).

6. Appareil de diagnostic par ultrasons (1A) selon l'une quelconque des revendications 3 à 5,
dans lequel, dans un cas où une pluralité d'opérations de balayage sont effectuées sur la même partie de plaie (J), l'unité de génération de cartes de probabilité (10) génère la carte de probabilité sur la base d'une pluralité de probabilités de présence calculées par l'unité de calcul de probabilité (9) correspondant à la pluralité d'opérations de balayage.

7. Appareil de diagnostic par ultrasons (1A) selon l'une quelconque des revendications 3 à 5,
dans lequel, dans un cas où une pluralité d'opérations de balayage sont effectuées sur la même partie de plaie (J), l'unité de génération de cartes de probabilité (10) génère la carte de probabilité pour chacune des opérations de balayage, intègre une pluralité de cartes de probabilité générées correspondant à la pluralité d'opérations de balayage, et affiche une carte de probabilité intégrée sur le moniteur (6).

8. Appareil de diagnostic par ultrasons (1A) selon l'une quelconque des revendications 3 à 7, comprenant en outre :
une unité de détection de région de défaillance (12) qui analyse la carte de probabilité ou l'image ultrasonore pour détecter une région de défaillance dans laquelle la carte de probabilité (M) n'est pas normalement générée ou l'image ultrasonore n'est pas normalement acquise,
dans lequel le moniteur (6) affiche la région de défaillance.

9. Appareil de diagnostic par ultrasons (1A) selon l'une quelconque des revendications 3 à 8, comprenant en outre :
une unité de détection de région balayée (14) qui détecte qu'une région (R1 à R4, T) a déjà été balayée sur la base des informations de position de la sonde ultrasonore (21) acquises par le capteur de position dans un cas où un balayage est effectué sur la partie de plaie ; et
une unité de notification (15) qui notifie un utilisateur dans un cas où il est détecté que la région a déjà été balayée.

10. Appareil de diagnostic par ultrasons (1A) selon l'une quelconque des revendications 3 à 9,
dans lequel le moniteur (6) affiche la carte de probabilité qui a déjà été générée par l'unité de génération de cartes de probabilité (10) sur la base des informations de position de la sonde ultrasonore (21) acquises par le capteur de position dans un cas où un balayage est effectué sur la partie de plaie (J).

11. Appareil de diagnostic par ultrasons (1A) selon l'une quelconque des revendications 3 à 10,
dans lequel l'unité de génération de cartes de probabilité (10) génère la carte de probabilité dans laquelle une région, qui a été balayée à plusieurs reprises un plus grand nombre de fois, a une couleur plus foncée ou une densité plus élevée.

12. Procédé de contrôle d'un appareil de diagnostic par ultrasons (1A), le procédé comprenant :
acquérir des informations de position d'une sonde ultrasonore (21) ;
balayer une partie de plaie (J) d'un sujet avec un faisceau ultrasonore en utilisant la sonde ultrasonore (21) pour acquérir des images ultrasonores d'une pluralité de trames ;
calculer une probabilité de présence d'une anomalie liée à la partie de plaie (J) à partir de chacune des images ultrasonores de la pluralité de trames, dans laquelle ladite anomalie est une structure et un schéma de tissus du sujet correspondant à des symptômes de décubitus tels que l'œdème, la nécrose et l'abcès ; et
générer une carte de probabilité tridimensionnelle (M) de l'anomalie sur la base des informations de position acquises de la sonde ultrasonore (21) et de la probabilité de présence calculée.

13. Processeur (22, 22A) pour un appareil de diagnostic par ultrasons (1A), le processeur (22, 22A) étant configuré pour :
acquérir des informations de position d'une sonde ultrasonore (21) ;
balayer une partie de plaie (J) d'un sujet avec un faisceau ultrasonore en utilisant la sonde ultrasonore (21) pour acquérir des images ultrasonores d'une pluralité de trames ;
calculer une probabilité de présence d'une anomalie liée à la partie de plaie (J) à partir de chacune des images ultrasonores de la pluralité de trames, dans laquelle ladite anomalie est une structure et un schéma de tissus du sujet correspondant à des symptômes de décubitus tels que l'œdème, la nécrose et l'abcès ; et
générer une carte de probabilité tridimensionnelle (M) de l'anomalie sur la base des informations de position acquises de la sonde ultrasonore (21) et de la probabilité de présence calculée.
